# EUROPÄISCHE PATENTSCHRIFT

(11) **EP 1 105 362 B1**
(45) Veröffentlichungstag und Bekanntmachung des Hinweises auf die Patenterteilung: **02.01.2003**
(21) Anmeldenummer: 99941607.6
(22) Anmeldetag: 13.08.1999
(51) Int. Cl.: C07C 29/42

(54) **VERFAHREN ZUR HERSTELLUNG VON ALKINDIOLEN**
METHOD FOR PRODUCING ALKYNE DIOLS
PROCEDE POUR LA PREPARATION DE DIOLS D'ALKINE

(30) Priorität: 17.08.1998 DE 19837211
(43) Veröffentlichungstag der Anmeldung: 13.06.2001
(73) Patentinhaber: BASF AKTIENGESELLSCHAFT, 67056 Ludwigshafen (DE)
(72) Erfinder: KINDLER, Alois, D-67165 Waldsee (DE); BRUNNER, Melanie, D-67105 Schifferstadt (DE); TRAGUT, Christian, D-67157 Wachenheim (DE); HENKELMANN, Jochem, D-68165 Mannheim (DE)
(74) Vertreter: Isenbruck, Günter, Dr.
(86) Internationale Anmeldenummer: EP9905933
(87) Internationale Veröffentlichungsnummer: WO00009465

(56) Entgegenhaltungen:
- DE-A- 2 008 675
- DE-A- 2 047 446

## Beschreibung

Die Erfindung betrifft ein Verfahren zur Herstellung von Alkindiolen durch Umsetzung von Ketonen mit acetylenischen Kohlenwasserstoffen unter Verwendung von Kaliumalkoholaten.

Für die Herstellung von Alkindiolen sind eine Reihe von Herstellungsverfahren bekannt.

Mit Hilfe des Reppeschen Ethinylierungsverfahrens, durch Umsetzung von Aldehyden wie Formaldehyd und Acetaldehyd mit Acetylen an Kupferacetylid als Katalysator, sind sekundäre Alkinmonoalkohole und Glycole in guten Ausbeuten erhältlich. Bei höheren Aldehyden führt diese Methode jedoch zu unbefriedigenden Ergebnissen.

Besonders problematisch ist die Herstellung von tertiären Alkindiolen durch Umsetzung von Ketonen mit acetylenischen Kohlenwasserstoffen unter Verwendung von Basen. Die meisten der bisher beschriebenen Verfahren verwenden fein verteiltes, möglichst wasserfreies KOH-Pulver in organischen Lösungsmitteln wie THF, Diisopropylether, Dioxan, Methylal oder Acetaldehyddibutylacetal. Nachteilig bei diesen Verfahren ist, daß in den meisten Fällen Gemische aus Monoalkinolen und Alkindiolen mit einem erheblichen Anteil an Monoalkinolen erhalten werden. Ein weiterer Nachteil ist, daß die in den genannten Lösungsmitteln entstehenden Suspensionen durch die Bildung nadelförmiger kristalliner Addukte aus KOH und tertiären Monoalkinolen und Alkindiolen so viskos werden, daß die Rührbarkeit erheblich erschwert wird. Dadurch ist eine gute Durchmischung und damit eine geregelte Abfuhr der Reaktionswärme erschwert bzw. nicht möglich. Das führt neben geringen Umsätzen auch zu sicherheitstechnischen Problemen. Eine mögliche Verwendung größerer Lösungsmittelmengen hat relativ geringe Wirkung auf das Viskositätsprofil und ist in der Regel nicht wirtschaftlich, da die verwendeten Lösungsmittel teuer sind.

In der EP-A 0 285 755 ist ein Verfahren zur Herstellung von tertiären Alkindiolen durch Umsetzung von Ketonen mit Acetylen beschrieben. Dabei wird Acetylen mit Carbonylverbindungen und KOH-Pulver als Base umgesetzt. Als Lösungsmittel werden Alkyl-tert.-butylether verwendet. Dabei werden das Keton und Acetylen in einem Molverhältnis von 1 : 1 bis 3 : 1 eingesetzt und KOH und Keton in einem Molverhältnis von 1 : 1 bis 1,6 : 1. Aufgrund des eingesetzten Lösungsmittels sollen gut rührbare Reaktionsgemische erhalten werden. Die Lehre dieser Anmeldung konnte jedoch nicht nachvollzogen werden (Vergleichsbeispiel 2). Nachteilig an diesem Verfahren ist außerdem der Einsatz von speziellen, teuren Lösungsmitteln, so daß das Verfahren nicht wirtschaftlich ist.

In der DE-A 20 08 675 wird die Herstellung tertiärer Alkindiole durch Umsetzung von Ketonen mit Acetylen unter Verwendung von Kaliumalkoholaten primärer und sekundärer Alkohole mit begrenzter Wasserlöslichkeit beschrieben. Als Lösungsmittel können aliphatische, cycloaliphatische und aromatische Kohlenwasserstoffe eingesetzt werden. Auch die DE-A 20 47 446 beschreibt die Verwendung von Kaliumalkoholaten zur Herstellung von Alkindiolen bei der Umsetzung von Alkinmonoalkohlen mit Ketonen.

In beiden Verfahren ist ein Ansteigen der Viskosität der Reaktionsmischung im Verlauf der Reaktion festzustellen. Eine gute Durchmischung des Reaktionsansatzes und die geregelte Abfuhr der Reaktionswärme sind daher erschwert, so daß die bereits erwähnten Probleme auch bei diesen Verfahren auftreten.

Aufgabe der vorliegenden Erfindung ist es daher, ein Verfahren zur Herstellung von Alkindiolen bereitzustellen, das mit einer wirtschaftlich vertretbaren Menge eines gebräuchlichen organischen Lösungsmittels auskommt. Die Reaktionsmischung soll während der gesamten Reaktionsdauer gut rührbar bleiben, so daß eine geregelte Abfuhr der Reaktionswärme und gute Umsätze gewährleistet sind.

Die Lösung dieser Aufgabe geht aus von einem Verfahren zur Herstellung von Alkindiolen durch Umsetzung von Ketonen mit acetylenischen Kohlenwasserstoffen, ausgewählt aus Acetylen und Alkinmonoalkoholen, in einem organischen Lösungsmittel in Gegenwart von Base, die Kaliumalkoholate primärer und/oder sekundärer Alkohole enthält, unter Bildung von aus der Reaktionsmischung ausfallenden Addukten aus Alkinmonoalkoholen und/oder Alkindiolen und Base. Das erfindungsgemäße Verfahren ist dann dadurch gekennzeichnet, daß bei einem Einsatz von Acetylen als acetylenischen Kohlenwasserstoff ein Verhältnis von eingesetztem Keton zu Acetylen von 1,9 bis 2,1 zu 1 und ein Verhältnis von Kaliumalkoholat zu dem eingesetzten Keton von 0,9 bis 2,1 zu 1 eingehalten wird und bei Einsatz von Alkinmonoalkoholen als acetylenischen Kohlenwasserstoffen ein Verhältnis von dem eingesetzten Alkinmonoalkohol zu dem eingesetzten Keton von 1 zu 0,8 bis 1,2 und ein Verhältnis von Kaliumalkoholat zu dem eingesetzten Keton von 1,5 bis 2,2 zu 1 eingehalten wird, so daß gelartige Addukte entstehen, die eine sphärische Oberfläche aufweisen, wodurch die Reaktionsmischung während der gesamten Umsetzung rührbar bleibt.

Bei den ausfallenden Addukten handelt es sich um Addukte der Base mit während der Umsetzung gebildeten Alkinmonoalkoholen oder Alkindiolen. Bei Einhaltung einer geeigneten Stöchiometrie der Reaktionspartner sind diese Addukte gelartig und nicht nadelförmig kristallin. Unter einer sphärischen Oberfläche ist im Sinne der Erfindung eine abgerundete, vorzugsweise kugelähnliche Oberfläche, wie sie in Addukten erfolgt sondern, daß die ausgefallenen Addukte beim Rühren aneinander vorbeigleiten können. Damit ist eine geregelte Abfuhr der Reaktionswärme und eine deutlich bessere Durchmischung der Reaktionspartner möglich. Neben einer vorteilhaften Auswirkung auf die Umsätze der Reaktion ist eine geregelte Abfuhr der Reaktionswärme auch aus sicherheitstechnischen Gründen erwünscht. Wenn diese nicht gewährleistet ist, kann es lokal zu einer Überschreitung der Zersetzungstemperatur der in der Reaktionsmischung vorhandenen Substanzen kommen und so zu spontanen Zersetzungen.

Unter acetylenischen Kohlenwasserstoffen sind im Sinne der vorliegenden Erfindung Acetylen und aus Carbonylverbindungen und Acetylen hergestellte Monoalkinole zu verstehen.

In dem erfindungsgemäßen Verfahren wird in einer Ausführungsform Acetylen als acetylenischer Kohlenwasserstoff eingesetzt. Aufgrund der guten Durchmischung wird die Stöchiometrie der Edukte dabei so gewählt, daß Acetylen stöchiometrisch bezüglich des Ketons eingesetzt wird. Dabei ist unter stöchiometrisch ein Verhältnis von Keton zu Acetylen von 1,9 : 1 bis 2,1 : 1, bevorzugt von 2 : 1 zu verstehen. Das Verhältnis von Kaliumalkoholat zu Keton beträgt 0,9 : 1 bis 2,1 : 1, bevorzugt 1 : 1 bis 1,5 : 1, besondes bevorzugt 1,1 : 1 bis 1,3 : 1. Das gewählte Verhältnis von Alkoholat zu Keton ist ein wesentlicher Faktor, um die Rührbarkeit der Reaktionsmischung zu gewährleisten, da bei dem gewählten Verhältnis keine nadelförmigen Addukte, sondern gelförmige Addukte mit einer sphärischen Oberfläche gebildet werden.

Die Konzentrationen der Reaktionspartner in der Reaktionsmischung können durch das Gewichtsverhältnis zwischen Keton und einer Suspension aus Lösungsmittel und Base angegebenen werden. Die Konzentration, bei der die Reaktionsmischung gut rührbar bleibt, ist abhängig von den Reaktionsbedingungen und insbesondere vom eingesetzten Keton, Lösungsmittel und Alkoholat. Bei Einsatz einer Suspension von Kalium-iso-Butylat in Xylol und Aceton beträgt das Gewichtsverhältnis zwischen Keton und der Suspension im allgemeinen mindestens 1 : 2,5, bevorzugt zwischen 1 : 2,5 bis 1: 8, besonders bevorzugt 1 : 6,5.

In einer weiteren Ausführungsform werden als acetylenische Kohlenwasserstoffe Alkinmonoalkohole eingesetzt. Die Herstellung der Alkinmonoalkohole durch Umsetzung von Acetylen mit Carbonylverbindungen kann nach in der Literatur bekannten Verfahren erfolgen.

Als Carbonylverbindungen können dabei aliphatische und aromatische Aldehyde und Ketone eingesetzt werden. Bevorzugt werden Ketone eingesetzt, wobei der Einsatz aliphatischer Ketone besonders bevorzugt ist. Diese können linear, verzweigt oder cyclisch sein. Bevorzugt werden Ketone mit 3 bis 8 Kohlenstoffatomen, besonders bevorzugt mit 3 bis 6 Kohlenstoffatomen eingesetzt, ganz besonders bevorzugt sind Aceton, Methylisobutylketon und Cyclohexanon.

Demgemäß werden als Acetylenmonoalkohole besonders bevorzugt Methyl-butinol, 3,5-Methyl-hex-1-in-3-ol und 3-Cyclohexyl-prop-1-in-3-ol eingesetzt.

Das Verhältnis von Alkinmonoalkohol zu Keton beträgt 1 : 0,8 bis 1 : 1,2, bevorzugt 1 : 1. Das Verhältnis von Kaliumalkoholat zu Keton beträgt 1,5 : 1 bis 2,2 : 1, bevorzugt bei 1,9 : 1 bis 2,1 : 1, besonders bevorzugt bei 2 : 1. Durch die gewählten Molverhältnisse werden die Rührbarkeit der Reaktionsmischung und somit gute Umsätze und eine geregelte Wärmeabfuhr ermöglicht.

Als Ketone können bei der Umsetzung von Ketonen mit acetylenischen Kohlenwasserstoffen aliphatische und aromatische Ketone eingesetzt werden. Bevorzugt ist der Einsatz von aliphatischen Ketonen. Diese können linear, verzweigt oder cyclisch sein. Besonders bevorzugt werden aliphatische Ketone mit 3 bis 8 Kohlenstoffatomen, ganz besonders bevorzugt mit 3 bis 6 Kohlenstoffatomen eingesetzt. Unter diesen sind Aceton, Methylisobutylketon und Cyclohexanon bevorzugt. Ganz besonders bevorzugt ist der Einsatz von Aceton.

Als Lösungsmittel sind insbesondere Kohlenwasserstoffe und Ether geeignet. Bevorzugt werden aliphatische und/oder aromatische Kohlenwasserstoffe eingesetzt. Besonders bevorzugt sind Kohlenwasserstoffe mit einem Siedebereich von 80 bis 180 °C. Ganz besonders bevorzugt werden aliphatische Kohlenwasserstoffe wie Benzingemische, cycloaliphatische Kohlenwasserstoffe wie Cyclohexan oder aromatische Kohlenwasserstoffe wie Benzol, Toluol, Xylol, Cumol oder p-Isopropylbenzol eingesetzt. Ganz besonders bevorzugt wird Xylol eingesetzt.

Als Kaliumalkoholate werden Kaliumalkoholate sekundärer und/oder primärer Alkohole eingesetzt. Dabei sind Alkoholate von C₃- bis C₈-Alkoholen, welche linear, verzweigt oder cyclisch sein können, bevorzugt.

Beispielsweise können die Alkoholate primärer Alkohole wie n-Butanol, Isobutanol, n-Pentanol, 2-Ethylbutanol-4, 2-Methylbutanol-1, 2,2-Dimethylpropanol-1, Hexanol, 2-Ethylhexanol sowie die Kaliumalkoholate sekundärer Alkohole wie Butanol-2, Pentanol-2, Pentanol-3, Methylbutanol-3 und Cyclohexanol eingsetzt werden. Besonders bevorzugt ist der Einsatz von Kaliumbutylaten, insbesondere von Kalium-iso-Butylat.

Ein Verfahren zur Gewinnung der Kaliumalkoholate ist in DE-A 20 08 675 beschrieben. Dazu wird wäßrige Kalilauge (z.B. eine 50 gew.-%ige wäßrige KOH-Lösung) mit überschüssigem Alkohol unter Rückflußkühlung zum Sieden erhitzt. Am Kopf einer Fraktionierkolonne wird aus dem sich bildenden zweiphasigen Azeotrop mit Wasser die untere wäßrige Schicht entfernt, der Alkohol aber wieder als Rücklauf der Kolonne zugeführt. In kurzer Zeit erhält man so eine Lösung des gewünschten Kaliumalkoholats. Nach Zugabe eines Kohlenwasserstoffs oder Ethers, der höher siedet als der verwendete Alkohol, wird der überschüssige Alkohol abdestilliert. Zurück bleibt ein alkoholfreies Kaliumalkoholat, daß im Kohlenwasserstoff zum Teil suspendiert, zum Teil gelöst ist.

Bei der Umsetzung von acetylenischen Kohlenwasserstoffen mit Carbonylverbindungen werden vorzugsweise alkoholfreie Kaliumalkoholate eingesetzt. Die Anwesenheit von Alkohol ist jedoch im allgemeinen nicht störend.

Als Base wird eine Mischung aus KOH und Kaliumalkoholat oder reines Kaliumalkoholat eingesetzt. Beispielsweise beträgt das Gewichtsverhältnis von KOH zu Kaliumbutylat vorzugsweise 30 : 70 bis 0 : 100 Gew.-%, besonders bevorzugt 5 : 95 bis 1 : 99 Gew.-% Ganz besonders bevorzugt ist ein Verhältnis von KOH zu Kaliumbutylat von 1: 99 Gew.-%.

Die Umsetzungstemperatur beträgt im allgemeinen 0 bis 50°C, bevorzugt 20 bis 30°C. Besonders bevorzugt wird die Umsetzung bei etwa 30°C durchgeführt.

In einer bevorzugten Ausführungsform wird zunächst eine Suspension aus Kaliumbase und Lösungsmittel hergestellt. Anschließend werden das Keton und Acetylen, bzw. das Keton und der Alkinmonoalkohol in dem entsprechenden Molverhältnis synchron in die Suspension aus Kaliumbase und Lösungsmittel eingeleitet. Die erhaltenen Reaktionsgemische können in üblichen Rührkesseln gut gerührt werden. Die Reaktionsdauer ist unter anderem abhängig von der eingesetzten Menge der Ausgangsstoffe. Sie beträgt beispielsweise bei Einsatz von 1 Mol Base zwischen 4 und 8 Stunden, bevorzugt 6 Stunden. Dabei werden das Keton und das Acetylen bzw. der Alkinmonoalkohol bevorzugt innerhalb von 4 Stunden synchron zudosiert und im Anschluß wird 2 Stunden nachgerührt. Nach Reaktionsende wird mit Wasser hydrolysiert, wobei die Base größtenteils als KOH in die wäßrige Phase übergeht. Durch Phasenabscheidung kann die entstehende wäßrige KOH-Lösung abgetrennt werden. Die Wertstoffe verbleiben in der organischen Phase und können nach Hydrolyse und anschließender Neutralisation, vorzugsweise mit Eisessig, destillativ isoliert werden. Das dabei abdestillierte Lösungsmittel kann wiederverwendet werden.

In dem erfindungsgemäßen Verfahren kann die Reaktionsmischung während der gesamten Umsetzung gut gerührt werden. Dadurch werden hohe Ausbeuten von im allgemeinen mindestens 70 %, bevorzugt mindestens 80 % erzielt.

Die folgenden Beispiele erläutern die Erfindung zusätzlich.

### Beispiele

### Beispiel 1 - Herstellung von Dimethylhexindiol:

In einen mit Rührer versehenen Doppelmantelreaktor werden 114 g Xylol und 112 g Kalium-*iso*-Butylat (1 mol) eingefüllt (c(Base)=5mol/l). Die Suspension wird auf 30°C temperiert und während der Reaktion bei dieser Temperatur gehalten. Parallel werden über 4 Stunden 58 g Aceton (1 mol) und 13 g Acetylen (0,5 mol) eingeleitet. Nach 2 h Nachreaktion wird mit 120 g Wasser hydrolysiert. Nach Abtrennung der organischen Phase und Neutralisation mit 7 g Eisessig lassen sich bei einem Umsatz von 98 % (bezogen auf Aceton) 61,5 g Dimethylhexindiol (entsprechend 86 % Ausbeute) isolieren. Daneben wird in 4 % Ausbeute der Alkinmonoalkohol Methylbutinol erhalten.

### Vergleichsbeispiel 1

In einen mit Rührer versehenen Doppelmantelreaktor werden 376 g Xylol und 97 g Kalium-*iso*-Butylat (0,87 mol) eingefüllt (c(Base)=1,8 mol/l). Die Suspension wird auf 30°C temperiert und während der Reaktion bei dieser Temperatur gehalten. Parallel werden über 4 Stunden 92 g Aceton (1,59 mol) und 20 g Acetylen (0,77 mol) eingeleitet. Nach 2 h Nachreaktion wird mit 105 g Wasser hydrolysiert. Nach Abtrennung der organischen Phase und Neutralisation mit 0,7 g Eisessig lassen sich bei einem Umsatz von 86 % (bezogen auf Aceton) 58,7 g Dimethylhexindiol (entsprechend 52 % Ausbeute isolieren). Daneben wird noch in 14,4 % Ausbeute der Alkinmonoalkohol Methylbutinol erhalten.

### Vergleichsbeispiel 2 (EP-A 0 285 755)

In einen mit Rührer versehenen Doppelmantelreaktor werden 350 g Methyl-tert.butylether und 74 g Kaliumhydroxid-Pulver (85 %) eingefüllt. Die Suspension wird auf 20°C temperiert und während der Reaktion bei dieser Temperatur gehalten. Parallel werden über 4 Stunden 69,9 g Aceton und 15,6 g Acetylen eingeleitet. Nach 1 h Nachreaktion wird mit 150 g Wasser hydrolysiert. Nach Abtrennung der organischen Phase und Neutralisation mit 13 g Eisessig lassen sich bei einem Umsatz von 92 % (bezogen auf Aceton) 61,3 g Dimethylhexindiol (entsprechend 72 % Ausbeute) isolieren. Der Reaktionsansatz wurde gegen Reaktionsende unrührbar fest.

### Beispiel 2 - Herstellung von Dimethylhexindiol via Methylbutinol:

In einen mit Rührer versehenen Doppelmantelreaktor werden 114 g Xylol und 112 g Kalium-*iso*-Butylat (1 mol) eingefüllt. Die Suspension wird auf 30°C temperiert und während der Reaktion bei dieser Temperatur gehalten. Parallel werden über 4 Stunden 29 g Aceton (0,5 mol) und 42 g Methylbutinol (0,5 mol) eingeleitet. Nach 2 h Nachreaktion wird mit 120 g Wasser hydrolysiert. Nach Abtrennung der organischen Phase und Neutralisation mit 13 g Eisessig lassen sich bei einem Umsatz von 98 % (bezogen auf Aceton) 58,7 g Dimethylhexindiol (entsprechend 83 % Ausbeute) isolieren.

### Vergleichsbeispiel 3

In einen mit Rührer versehenen Doppelmantelreaktor werden 114 g Xylol und 112 g Kalium-*iso*-Butylat (1 mol) eingefüllt. Die Suspension wird auf 30°C temperiert und während der Reaktion bei dieser Temperatur gehalten. Parallel werden über 4 Stunden 55,1 g Aceton (0,95 mol) und 80 g Methylbutinol (0,95 mol) eingeleitet. Nach 2 h Nachreaktion wird mit 120 g Wasser hydrolysiert. Nach Abtrennung der organischen Phase und Neutralisation mit 21 g Eisessig lassen sich bei einem Umsatz von 93 % (bezogen auf Aceton) 93 g Dimethylhexindiol (entsprechend 69 % Ausbeute) isolieren. Daneben wird in 14,3 % Ausbeute der Alkinmonoalkohol Methylbutinol erhalten.

### Beispiel 3 - Herstellung von 4,7-Dihydroxy-2,4,7,9-tetramethyl-hex-5-in:

In einen mit Rührer versehenen Doppelmantelreaktor werden 310 g Xylol und 224 g Kalium-*iso*-Butylat (2 mol) eingefüllt. Die Suspension wird auf 30°C temperiert und während der Reaktion bei dieser Temperatur gehalten. Parallel werden über 4 Stunden 200 g Methylisobutylketon (2 mol) und 26 g Acetylen (1 mol) eingeleitet.

Nach 2 h Nachreaktion wird mit 230 g Wasser hydrolysiert. Nach Abtrennung der organischen Phase und Neutralisation mit Eisessig lassen sich bei einem Umsatz von 89 % (bezogen auf Aceton) 330 g 4,7 Dihydroxy-2,4,7,9-tetramethyl-hex-5-in (entsprechend 73 % Ausbeute isolieren).

### Konzentrationsabhängigkeit der Selektivität der Umsetzung von Aceton mit Acetylen zu Dimethylhexindiol unter Einsatz einer Suspension von Kalium-iso-Butylat in Xylol bei verschiedenen Gewichtsverhältnissen zwischen der K-Base in Xylol und Aceton

### Beispiel 4 - Herstellung von Dimethylhexindiol (K-Base in Xylol/Aceton=6,4)

In einen mit Rührer versehenen Doppelmantelreaktor werden 228 g Xylol und 112 g Kalium-*iso*-Butylat eingefüllt. Die Suspension wird auf 30°C temperiert und während der Reaktion bei dieser Temperatur gehalten. Parallel werden über 4 Stunden 53,3 g Aceton und 11,7 g Acetylen eingeleitet. Nach 2 h Nachreaktion wird mit 120 g Wasser hydrolisiert. Nach Abtrennung der organischen Phase und Neutralisation mit 13 g Eisessig lassen sich bei einem Umsatz von 98 % (bezogen auf Aceton) 57,1 g Dimethylhexindiol (entsprechend 88 % Ausbeute) isolieren. Daneben wird in 7 % Ausbeute der Alkinmonoalkohol Methylbutinol erhalten.

### Beispiel 5 - Herstellung von Dimethylhexindiol (K-Base in Xylol/Aceton=4,5):

In einen mit Rührer versehenen Doppelmantelreaktor werden 114 g Xylol und 112 g Kalium-*iso*-Butylat eingefüllt. Die Suspension wird auf 30°C temperiert und während der Reaktion bei dieser Temperatur gehalten. Parallel werden über 4 Stunden 53,3 g Aceton und 11,7 g Acetylen eingeleitet. Nach 2 h Nachreaktion wird mit 120 g Wasser hydrolysiert. Nach Abtrennung der organischen Phase und Neutralisation mit 11 g Eisessig lassen sich bei einem Umsatz von 98 % (bezogen auf Aceton) 56,7 g Dimethylhexindiol (entsprechend 87 % Ausbeute) isolieren.

Daneben wird in 7 % Ausbeute der Alkinmonoalkohol Methylbutinol erhalten.

### Beispiel 6 - Herstellung von Dimethylhexindiol (K-Base in Xylol/Aceton=3,6):

In einen mit Rührer versehenen Doppelmantelreaktor werden 80 g Xylol und 112 g Kalium-*iso*-Butylat eingefüllt. Die Suspension wird auf 30°C temperiert und während der Reaktion bei dieser Temperatur gehalten. Parallel werden über 4 Stunden 53,5 g Aceton und 11,7 g Acetylen eingeleitet. Nach 2 h Nachreaktion wird mit 120 g Wasser hydrolysiert. Nach Abtrennung der organischen Phase und Neutralisation mit 15 g Eisessig lassen sich bei einem Umsatz von 98 % (bezogen auf Aceton) 51,8 g Dimethylhexindiol (entsprechend 79 % Ausbeute) isolieren. Daneben wird in 5 % Aus-beute der Alkinmonoalkohol Methylbutinol erhalten.

### Beispiel 7 - Herstellung von Dimethylhexindiol (K-Base in Xylol/Aceton=3,2)

In einen mit Rührer versehenen Doppelmantelreaktor werden 114 g Xylol und 224 g Kalium-*iso*-Butylat eingefüllt. Die Suspension wird auf 30°C temperiert und während der Reaktion bei dieser Temperatur gehalten. Parallel werden über 4 Stunden 107 g Aceton und 23,4 g Acetylen eingeleitet. Nach 2 h Nachreaktion wird mit 240 g Wasser hydrolysiert. Nach Abtrennung der organischen Phase und Neutralisation mit 18 g Eisessig lassen sich bei einem Umsatz von 98 % (bezogen auf Aceton) 91,6 g Dimethylhexindiol (entsprechend 70 % Ausbeute) isolieren. Daneben wird in 4 % Ausbeute der Alkinmonoalkohol Methylbutinol erhalten.

## Patentansprüche

1. Verfahren zur Herstellung von Alkindiolen durch Umsetzung von Ketonen mit acetylenischen Kohlenwasserstoffen, ausgewählt aus Acetylen und Alkinmonoalkoholen, in einem organischen Lösungsmittel in Gegenwart von Base, die Kaliumalkoholate primärer und/oder sekundärer Alkohole enthält, unter Bildung von aus der Reaktionsmischung ausfallenden Addukten aus Alkinmonoalkoholen und/oder Alkindiolen und Base, **dadurch gekennzeichnet, daß** bei einem Einsatz von Acetylen als acetylenischen Kohlenwasserstoff ein Verhältnis von eingesetztem Keton zu Acetylen von 1,9 bis 2,1 zu 1 und ein Verhältnis von Kaliumalkoholat zu dem eingesetzten Keton von 0,9 bis 2,1 zu 1 eingehalten wird und bei Einsatz von Alkinmonoalkoholen als acetylenischen Kohlenwasserstoffen ein Verhältnis von dem eingesetzten Alkinmonoalkohol zu dem eingesetzten Keton von 1 zu 0,8 bis 1,2 und ein Verhältnis von Kaliumalkoholat zu dem eingesetzten Keton von 1,5 bis 2,2 zu 1 eingehalten wird, so daß gelartige Addukte entstehen, die eine sphärische Oberfläche aufweisen, wodurch die Reaktionsmischung während der gesamten Umsetzung rührbar bleibt.

2. Verfahren nach Anspruch 1, **dadurch gekennzeichnet, daß**, bei Einsatz von Acetylen als acetylenischen Kohlenwasserstoff, Acetylen in stöchiometrischer Menge bezüglich des Ketons eingesetzt wird und das Verhältnis von Kaliumalkoholat zu Keton 1 : 1 bis 1,5 : 1 beträgt.

3. Verfahren nach Anspruch 2, **dadurch gekennzeichnet, daß** das Verhältnis von Kaliumalkoholat zur Keton 1,1 : 1 bis 1,3 : 1 beträgt.

4. Verfahren nach Anspruch 1, **dadurch gekennzeichnet, daß**, bei Einsatz eines Alkinmonoalkohols als acetylenischen Kohlenwasserstoff, das Verhältnis von Alkinmonoalkohol zu Keton 1 : 1 und das Verhältnis von Kaliumalkoholat zu Keton 1,9 : bis 2,1 : 1 beträgt.

5. Verfahren nach einem der Ansprüche 1 bis 4, **dadurch gekennzeichnet, daß** Ketone ausgewählt aus Aceton, Methylisobutylketon und Cyclohexanon eingesetzt werden.

6. Verfahren nach Anspruch 5, **dadurch gekennzeichnet, daß** als Keton Aceton eingesetzt wird.

7. Verfahren nach einem der Ansprüche 1 bis 6, **dadurch gekennzeichnet, daß** als Lösungmittel Kohlenwasserstoffe eingesetzt werden.

8. Verfahren nach einem der Ansprüche 1 bis 7, **dadurch gekennzeichnet, daß** als Kaliumalkoholate Kaliumbutylate eingesetzt werden.

## Claims

1. A process for preparing alkynediols by reacting ketones with acetylenic hydrocarbons selected from acetylene and alkynemonools in an organic solvent in the presence of a base comprising potassium alkoxides of primary and/or secondary alcohols to form adducts of alkynemonools and/or alkynediols and said base which precipitate from the reaction mixture, which comprises, when using acetylene as acetylenic hydrocarbon, observing a ratio of ketone to acetylene of from 1.9 : 1 to 2.1 : 1 and a ratio of potassium alkoxide to ketone of from 0.9 : 1 to 2.1 : 1 and, when using alkynemonools as acetylenic hydrocarbons, observing a ratio of alkynemonool to ketone of from 1 : 0.8 to 1 : 1.2 and a ratio of potassium alkoxide to ketone of from 1.5 : 1 to 2.2 : 1, so as to produce gellike adducts having a spherical surface, whereby the reaction mixture remains stirrable during the entire reaction.

2. A process as claimed in claim 1, wherein, when acetylene is used as acetylenic hydrocarbon, acetylene is used in a stoichiometric amount with regard to the ketone and the ratio of potassium alkoxide to ketone is within the range from 1 : 1 to 1.5 : 1.

3. A process as claimed in claim 2, wherein the ratio of potassium alkoxide to ketone is within the range from 1.1 : 1 to 1.3 : 1.

4. A process as claimed in claim 1, wherein, when an alkynemonool is used as acetylenic hydrocarbon, the ratio of alkynemonool to ketone is within the range from 1 : 1 and the ratio of potassium alkoxide to ketone is within the range from 1.9 : 1 to 2.1 : 1.

5. A process as claimed in any of claims 1 to 4, wherein ketones selected from acetone, methyl isobutyl ketone and cyclohexanone are used.

6. A process as claimed in claim 5, wherein acetone is used as ketone.

7. A process as claimed in any of claims 1 to 6, wherein hydrocarbons are used as solvents.

8. A process as claimed in any of claims 1 to 7 wherein the potassium alkoxides used are potassium butoxides.

## Revendications

1. Procédé de préparation d'alcynediols par réaction de cétones avec des hydrocarbures acétyléniques, choisis parmi l'acétylène et des aminoalcools, dans un solvant organique en présence d'une base, contenant des alcoolates de potassium et / ou des alcools secondaires, avec formation de produits d'addition qui précipitent du mélange réactionnel et sont constitués de monoalcools d'alcyne et / ou d'alcynediols et de base, **caractérisé en ce que**, lorsque l'on met en oeuvre de l'acétylène comme hydrocarbure acétylénique, on respecte une proportion de la cétone mise en oeuvre à l'acétylène de 1,9 - 2 à 1 et une proportion d'alcoolate de potassium à la cétone mise en oeuvre de 0,9 - 2,1 à 1 et que, lorsque l'on met en oeuvre des monoalcools d'alcyne comme hydrocarbures acétyléniques, on respecte une proportion du monoalcool d'alcyne mis en oeuvre à la cétone mise en oeuvre de 1 à 0,8 - 1,2 et une proportion d'alcoolate de potassium à la cétone mise en oeuvre de 1,5 - 2,2 à 1, de telle manière qu'il se forme des produits d'addition gélatineux, présentant une surface sphérique, de sorte qu'il demeure possible de brasser le mélange réactionnel pendant toute la durée de la réaction.

2. Procédé selon la revendication 1, **caractérisé en ce que**, lorsque l'on met en oeuvre de l'acétylène comme hydrocarbure acétylénique, l'acétylène est mise en oeuvre en quantité stoechiométrique vis-à-vis de la cétone et que la proportion d'alcoolate de potassium à la cétone est de 1 : 1 à 1,5 : 1.

3. Procédé selon la revendication 2, **caractérisé en ce que** la proportion d'alcoolate de potassium à la cétone est de 1,1 : 1 à 1,3:1.

4. Procédé selon la revendication 1, **caractérisé en ce que**, lorsque l'on met en oeuvre un monoalcool d'amine comme hydrocarbure acétylénique, la proportion de monoalcool d'aminé à la cétone est de 1 : 1 et la proportion d'alcoolate de potassium à la cétone est de 1,9 : 1 à 2,1 : 1.

5. Procédé selon l'une des revendications 1 à 4, **caractérisé en ce que** la cétone est choisie parmi l'acétone, la méthyl-isobutylcétone et la cyclohexanone.

6. Procédé selon la revendication 5, **caractérisé en ce que** l'on met en oeuvre de l'acétone en tant que cétone.

7. Procédé selon l'une des revendications 1 à 6, **caractérisé en ce que** l'on utilise des hydrocarbures comme solvants.

8. Procédé selon l'une des revendications 1 à 7, **caractérisé en ce que** l'on utilise comme alcoolates de potassium des butylates de potassium.
